# EUROPÄISCHE PATENTANMELDUNG

(11) **EP 3 437 592 A1**
(43) Veröffentlichungstag der Anmeldung: **06.02.2019**
(21) Anmeldenummer: 18186777.1
(22) Anmeldetag: 01.08.2018
(51) Int. Cl.: A61F 5/01, A61F 5/37

(54) **HYGIENESPREIZER ZUM AUSEINANDERHALTEN DER BEINE EINES MENSCHEN**

(30) Priorität: 04.08.2017 DE 202017104691 U; 29.09.2017 DE 102017122815; 26.10.2017 DE 102017125048
(71) Anmelder: Kühl, Klaus, 25336 Elmshorn (DE); Vosteen, Jens, 25578 Neuenbrook (DE)
(72) Erfinder: Kühl, Klaus, 25336 Elmshorn (DE); Vosteen, Jens, 25578 Neuenbrook (DE)
(74) Vertreter: Jaeschke, Rainer

(57) **Zusammenfassung**

Die Erfindung betrifft einen Hygienespreizer zum Auseinanderhalten der Beine eines Menschen insbesondere während der Pflege. Gemäß der Erfindung wird vorgeschlagen, dass der Hygienespreizer an seinen gegenüberliegenden Enden jeweils eine Stützschale aufweist, die in der Gebrauchslage jeweils an der Innenseite eines Beins des Menschen anliegt, und dass zwischen den zugwandten Seiten der Stützschalen ein Stellmittel vorhanden ist, um die Stützschalen zwischen einer engen Gebrauchslage, in der die Stützschalen einen kleinen Abstand zueinander aufweisen, und einer weiten Gebrauchslage, in der die Stützschalen einen großen Abstand zueinander aufweisen, hin- und her zu bewegen.

## Beschreibung

Die Erfindung betrifft einen Hygienespreizer zum Auseinanderhalten der Beine eines Menschen während der Pflege. Insbesondere sollen die Beine des Menschen durch den Hygienespreizer temporär auseinander gehalten werden können.

Es ist bekannt, dass bettlägerige und/oder behinderte Menschen sich in der Regel nicht selbst waschen können. Es ist daher erforderlich, dass die Körperpflege durch Pflegepersonal durchgeführt wird. Es ist dabei unvermeidlich, dass das Pflegepersonal den Körper der betreffenden Person berühren muss. Solche Berührungen führen insbesondere bei behinderten Personen häufig zu Spastiken, durch die sich die betreffende Person ungewollt verkrampft. Bei einer solchen Verkrampfung werden die Beine fest zusammengepresst, so dass eine Pflege zwischen den Beinen und im Gesäßbereich nicht möglich oder zumindest erheblich erschwert wird.

Grundsätzlich ist es möglich, dass die Beine der zu pflegenden Person manuell auseinander gehalten werden. Hierfür werden jedoch zwei Personen benötigt, um zum einen die Beine auseinander zu halten und zum anderen die Person zu waschen. Dies erfordert mehr Pflegepersonal, das häufig und insbesondere in der häuslichen Pflege nicht zur Verfügung steht. Auch ist das Auseinanderdrücken der Beine während eines Krampfes für die zu pflegende Person mit Schmerzen verbunden.

Der Erfindung liegt die Aufgabe zugrunde, einen Hygienespreizer zum Auseinanderhalten der Beine eines Menschen während der Pflege zu schaffen, der einfach anzulegen und zu gebrauchen ist.

Die Aufgabe wird gemäß der Erfindung dadurch gelöst, dass der Hygienespreizer an seinen gegenüberliegenden Enden jeweils eine Stützschale aufweist, die in der Gebrauchslage jeweils an der Innenseite eines Beines des Menschen anliegt, und dass zwischen den zugwandten Seiten der Stützschalen ein Stellmittel vorhanden ist, um die Stützschalen zwischen einer engen Gebrauchslage, in der die Stützschalen einen kleinen Abstand zueinander aufweisen, und einer weiten Gebrauchslage, in der die Stützschalen einen großen Abstand zueinander aufweisen, hin- und her zu bewegen. Durch diese einfache Vorrichtung ist es möglich, die Beine eines Menschen auseinander zu halten. In der gespreizten Lage der Beine können deren Innenseite und auch der Gesäßbereich gut gewaschen werden. Vorzugsweise liegen die Stützschalen im Bereich des Knies an dem Bein an.

Die Stützschalen können als auf ihren abgekehrten Seiten offene Halbschalen ausgebildet sein, die in der Gebrauchslage an den Innenseiten der Oberschenkel oberhalb des Knies oder an den Innenseiten der Unterschenkel unterhalb des Knies anliegen. Auch ist es möglich, dass die Stützschalen sich von der Innenseite des Unterschenkels zur Innenseite des Oberschenkels über das Knie hinweg erstrecken und somit den gesamten Bereich des Knies abdecken.

Weiterhin ist es möglich, dass auf einer Seite des Hygienespreizers zwei Stützschalen vorhanden sind, von denen eine an der Innenseite des Oberschenkels oberhalb des Knies und die andere an der Innenseite des Unterschenkels unterhalb des Knies anliegt. Das Knie selbst bleibt dann frei, und der empfindliche Kniebereich wird nicht von den Stützschalen berührt.

Die Stützschalen oder Halbschalen oder Halteschalen können dabei quer zu der Schalenkrümmung gerade ausgebildet sein oder eine gekrümmte Form haben, damit sie sich gut an die Kontur des Beins anpassen. Es ist zweckmäßig, wenn zumindest die dem Bein zugewandte Seite der Stützschalen mit einem elastischen- und/der weichen Material oder einer Polsterung versehen ist. Hierdurch werden Druckstellen oder Verletzungen des Menschen im Falle einer Verkrampfung vermieden. Gemäß einer einfachen Ausbildung der Erfindung können die Stützschalen aber auch als einfache ebene Platten ausgebildet sein.

Gemäß der Erfindung ist weiterhin vorgesehen, dass das elastische und/oder weiche Material Bestandteil eines Bezugs ist, der über die Stützschalen ziehbar ist. Hierdurch wird erreicht, dass das mit der Haut der zu pflegenden Person in Kontakt kommende Material gereinigt werden kann. Alternativ kann die Polsterung fest mit der Stützschale verbunden und abwaschbar sein. Hier kann ein Stoffbezug vorgesehen werden, der abnehmbar und waschbar ist. Dadurch bleibt der Hygienespreizer hygienisch sauber und kann für mehrere Personen benutzt werden.

Für die Pflege eines Menschen wird der Hygienespreizer vor dem Waschen der empfindlichen Beininnenseite und des empfindlichen Gesäßbereichs zwischen die Beine im Bereich des Knies angelegt. Im Falle einer Verkrampfung während des Waschvorgangs stützen sich die Beine an den Stützschalen ab und werden durch das Stellmittel in der gespreizten Lage gehalten. Der Waschvorgang kann ohne weiteres fortgesetzt werden. Nach der Beendigung des Waschvorgangs kann der Hygienespreizer wieder entfernt werden.

Es ist günstig, wenn an den Stützschalen Riemen befestigt sind, um die Stützschale in der Gebrauchslage an dem betreffenden Bein zu fixieren. Hierdurch wird der Hygienespreizer unverrückbar zwischen den Beinen der Person gehalten, so dass im Falle einer auftretenden Spastik die Beine sicher auseinander gehalten werden.

Gemäß einer weitergehenden Ausführungsform der Erfindung ist vorgesehen, dass eine oder beide Stützschalen um wenigstens eine Achse gelenkig mit dem Stellmittel verbunden sind. Zweckmäßig ist es hierbei, dass eine oder beide Stützschalen über ein Kugelgelenk mit dem Stellmittel verbunden sind. Durch diese dreh- und schwenkbare Halterung der Stützschalen relativ zum Stellmittel, liegen die Stützschalen unabhängig von der Winkellage der zu haltenden Beine, vollflächig an diesen an. Dadurch werden Druckstellen vermieden und ein angenehmes Anlegen des Hygienespreizers wird erreicht.

Es kann weiterhin vorgesehen werden, dass eine oder beide Stützschalen lösbar mit dem Stellmittel verbunden sind. Damit ist es möglich, den Hygienespreizer mit unterschiedlichen Stützschalen zu versehen, um den Hygienespreizer an unterschiedlich große Beine anpassen zu können.

Gemäß einer besonders bevorzugten Ausführungsform der Erfindung ist vorgesehen, dass die Stützschalen entgegen der Kraft einer Feder von einer weiten Gebrauchslage in Richtung auf die enge Gebrauchslage bewegbar sind. Damit werden die Beine der zu pflegenden Person elastisch und sanft auseinander gedrückt. Bei einer Verkrampfung drücken die Beine nicht gegen einen starren Widerstand, sondern die Bewegung wird federnd aufgefangen. Dadurch werden Schmerzen oder Verletzungen bei einer Spastik vermieden.

Durch das Vorsehen einer Feder sind die Stützschalen nicht starr sondern federnd miteinander über das Stellmittel miteinander verbunden. Insbesondere bleiben die Stützschalen in ihrer weiten Gebrauchslage aufgrund der Feder gegeneinander beweglich. Eine auftretende Spastik wird daher federnd aufgefangen.

Weiterhin ist es günstig, wenn das Stellmittel in der voll ausgefahrenen Position, die der weiten Gebrauchslage der Stützschalen entspricht, und/oder der voll eingefahrenen Position, die der engen Gebrauchslage der Stützschalen entspricht, und/oder in mehreren Positionen zwischen der ausgefahrenen und eingefahren Position arretierbar ist. Dies hat den Vorteil, dass der Abstand der Stützschalen in einer günstigen Länge für die zu pflegende Person einerseits oder das Pflegepersonal andererseits voreinstellbar ist.

Es kann vorgesehen sein, dass der Abstand der Stützschalen in der engen Gebrauchslage zwischen 7 cm und 20 cm und in der weiten Gebrauchslage zwischen 20 cm und 60 cm beträgt. Durch diesen geringen Abstand der Stützschalen in der engen Gebrauchslage kann der Hygienespreizer auch bei zusammengepressten Beinen gut angelegt werden.

Gemäß einer Ausführungsform der Erfindung ist das Stellmittel als Teleskopstange ausgebildet. Die Teleskopstange kann als Federstange ausgebildet sein derart, dass die Stützschalen entgegen der Kraft einer Feder zusammendrückbar sind und aufgrund der Federkraft in der weiten Gebrauchslage gehalten werden. Die Teleskopstange kann eine Gasdruckfeder sein. Durch die variable Länge kann die Stange und somit den Hygienespreizer an unterschiedlich großen Personen oder unterschiedliche Beweglichkeiten der Personen angepasst werden.

Besonders zweckmäßig ist es, wenn die Stange in der voll ausgefahrenen und/oder der voll eingefahrenen Position und/oder in mehreren Positionen zwischen der kürzesten und der längsten Länge arretierbar ist. Dies hat den Vorteil, dass die Stange in einer günstigen Länge für die zu pflegende Person einerseits oder das Pflegepersonal andererseits voreinstellbar ist.

Für eine bessere Handhabung der Vorrichtung kann auch vorgesehen werden, dass die Feder auf einer Halterung am Stellmittel hin- und herbewegbar gehalten ist. Die Feder braucht dann nur einen geringen Federweg aufzuweisen. Der Federweg braucht beispielsweise nur 5,0 cm bis 10,0 cm betragen. Die Halterung ist in Bewegungsrichtung der Stützschalten am Stellmittel verschiebbar gehalten. Hierdurch wird erreicht, dass sich die Stützschalen von der engen Gebrauchslage nicht ruckartig in die weite Gebrauchslage bewegen, wenn die Arretierung, die die Stützschalen zum Anlegen der Vorrichtung in der engen Gebrauchslage hält, gelöst wird. Hier ist zu beachten, dass die Feder relativ stark sein muss, da bei einer auftretenden Spastik sehr hohe Kräfte freigesetzt werden. Eine "weiche" Feder würd dann vollkommen zusammengedrückt werden. Erst durch die richtige Wahl einer "strammen" Feder kann die Spastik relativ gut abgefedert werden.

Da der Federweg demnach auf den relativen kurzen Weg begrenzt ist, braucht das Pflegepersonal die Stützschalen vor dem Anlegen nicht mehr entgegen der Feder über den gesamten Spreizweg zusammenzudrücken und in der engen Gebrauchslage zu arretieren. Vielmehr reicht es aus, die Halterung in die enge Gebrauchslage zu bewegen und dort vorzugsweise zu arretieren. Dann wird die Vorrichtung zwischen den Beinen des Patienten angelegt, und die Halterung wird bis zu einer nächsten Arretierung bewegt, so dass die Beine auseinander bewegt werden. Da die Feder stets frei zusammendrückbar bleibt, wird sie bei einer auftretenden Spastik des Patienten zusammengedrückt, so dass die Spastik relativ schmerzfrei aufgefangen wird, ohne dass sich die Beine krampfartig zusammenpressen. Der relativ kurze Federweg der "strammen" Feder reicht hierfür aus.

Durch die Ausbildung des Stellmittels als Federstange können die Stützschalen entgegen der Kraft einer Feder zusammendrückbar sein. Aufgrund der Federkraft werden die Stützschalen selbsttätig in der ausgefahrenen Gebrauchslage gehalten. Es kann dabei vorgesehen werden, dass die Feder in der Stange angeordnet ist. Auch ist es möglich, dass das Stellmittel eine Gasdruckfeder ist.

Die Ausbildung des Stellmittels als Federstange hat weiterhin den Vorteil, dass der Hygienespreizer in der eingefahrenen Lage relativ einfach zwischen den Beinen der zu pflegenden Person angelegt werden kann. Zum weiteren Spreizen der Beine für die Pflege wird die vorzugsweise vorhandene Arretierung gelöst, so dass die Feder die Beine auseinander drückt. Dabei kann die maximale Länge durch entsprechende Anschläge eingestellt werden. Insbesondere kann die Länge der Stange so gewählt werden, dass ein bequemes Waschen der zu pflegenden Person möglich ist.

Nach dem Gebrauch wird die Stange wieder eingefahren und entfernt. Der Waschvorgang kann durch die Stange von einer

Person in relativ kurzer Zeit gut durchgeführt werden. Die Beine der zu pflegenden Person werden auch im Falle einer Spastik sanft auseinander gehalten, ohne dass die zu pflegende Person Schmerzen oder Verletzungen erleidet.

Der Hygienespreizer kann vorzugsweise aus Edelstahl oder einem stabilen Kunststoff bestehen. Die Wahl dieses Material hat den Vorteil, dass der Hygienespreizer gegen das Waschwasser unempfindlich ist. Die Bezüge der Stützschalen sind abziehbar, so dass die Bezüge unabhängig von dem Hygienespreizer gereinigt werden können. Es kann dabei vorgesehen werden, dass das Pflegepersonal einen Hygienespreizer mit mehreren unterschiedlich großen Stützschalen und/oder unterschiedlichen Bezügen ausgerüstet ist, so dass der Hygienespreizer für mehrere zu pflegende Personen verwendbar ist.

Gemäß einer anderen Ausführungsform der Erfindung ist das Stellmittel als Scherengelenk ausgebildet, dessen Scherenarme über eine Stellschraube zwischen der ausgefahrenen und eingefahrenen Position hin- und herbewegbar sind. Ein Scherengelenk ermöglicht eine enge Gebrauchslage mit einem relativ kleinen Abstand der Stützschalen zueinander. Dadurch kann der Hygienespreizer auch bei zusammengepressten Beinen relativ gut zwischen die Oberschenkel der betreffenden Person positioniert werden.

Es kann vorgesehen werden, dass die Stützschalen an der Verbindungstelle an den auseinander gehenden Enden der Scherenarme angelenkt sind. Die Scherenarme können unmittelbar mit nur einem Gelenk miteinander verbunden sein. Auch ist es möglich zwischen den auseinandergehenden Enden der Scherarme eine Verbindungsplatte vorzusehen, die jeweils gelenkig mit den auseinandergehenden Enden der Scherenarme verbunden ist. Diese Verbindungsplatten bewegen sich dann mit parallelen Oberflächen aufeinander zu oder voneinander weg, so dass die Stützschalen gut auf den Verbindungsplatten vorzugsweise über ein Kugelgelenk gehalten werden können.

Gemäß einer weitergehenden Ausführungsform der Erfindung ist vorgesehen, dass das Stellmittel über eine Haltevorrichtung an einem Stuhl des Menschen befestigbar ist. Dieser Stuhl kann ein Rollstuhl sein. Dann kann der Hygienespreizer gut für die angenehme Haltung des Menschen während des Sitzens verwendet werden. Der Hygienespreizer verhindert ein unkontrolliertes und unbequemes Zusammenpressen der Beine, so dass die Person relativ entspannt auf dem Stuhl sitzen kann. Da das Haltemittel an dem Stellmittel angreift, bleiben die Halteschalen gegeneinander beweglich, so dass deren Funktion aufrechterhalten bleibt.

Dabei ist es günstig, wenn die Haltevorrichtung ein Gestänge umfasst, deren einzelne Stangen über feststellbare Gelenke miteinander verbunden sind. Die Gelenke sind vorzugsweise verschiebbar auf den Stangen gelagert. Dann kann der Hygienespreizer an unterschiedliche Sitzpositionen oder Personen angepasst werden.

Die Erfindung wird im Folgenden anhand der schematischen Zeichnung näher erläutert. Es zeigen:
- Fig. 1: einen Hygienespreizer in der Gebrauchslage,
- Fig. 2: eine andere Ausführungsform des Hygienespreizers,
- Fig. 3: eine weitere Ausführungsform des Hygienespreizers und
- Fig. 4: einen Hygienespreizer mit einer Haltevorrichtung.

In der Zeichnung sind die auseinander zu haltenden Oberschenkel 11 eines Menschen als Kreise dargestellt. Gezeigt ist der Oberschenkel oder Unterschenkel eines Beins im Bereich oberhalb beziehungsweise unterhalb des Knies. Die beiden Beine 11 sollen durch den Hygienespreizer 12 auseinander gehalten werden.

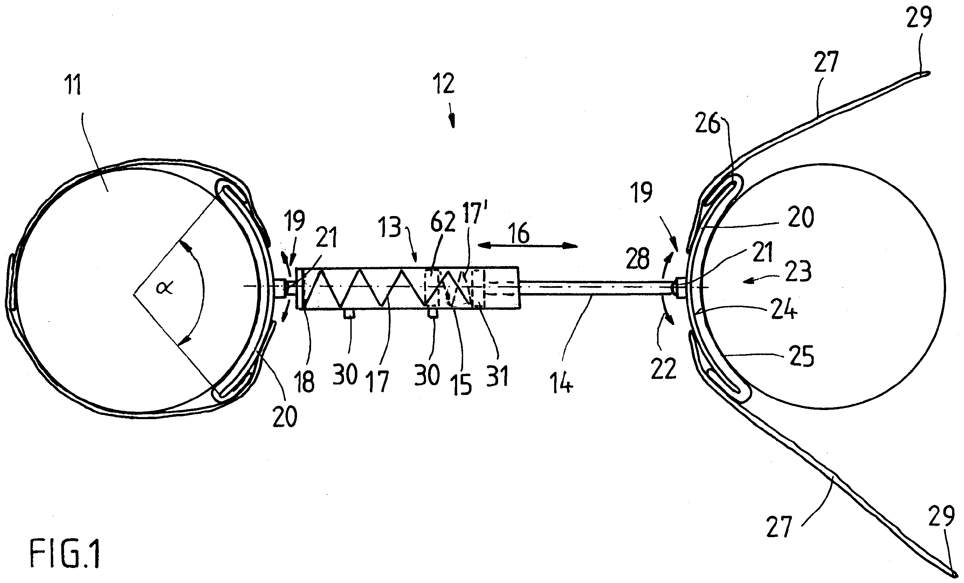

Der Hygienespreizer 12 umfasst ein Stellmittel 13, das bei dem Ausführungsbeispiel in Figur 1 als Federstange ausgebildet ist. Die Federstange weist einen Kolben 14 auf, der in einem Rohr 15 teleskopartig in Richtung des Doppelpfeils 16 hin- und her bewegbar gelagert ist. Das im Zylinderrohr 15 liegende Ende der Kolbenstange 14 weist bei dem dargestellten Ausführungsbeispiel eine Scheibe 31 auf, deren Außendurchmesser dem Innendurchmesser des Rohres 15 entspricht. Auf der dem Kolben abgekehrten Seite der Scheibe 31 ist eine Feder 17 vorhanden, die sich einerseits an der Scheibe 31 und andererseits an der dem Hubkolben 14 abgewandten inneren Stirnseite 18 des Zylinders 15 abstützt. Die Schraubenfeder 17 ist als Druckfeder ausgebildet derart, dass die Federkraft den Hubkolben 14 aus dem Zylinder hinaustreibt. Demnach kann der Kolben 14 entgegen der Kraft der Feder 17 in den Zylinder hineinbewegt werden, wodurch sich die Länge der Federstange 14, 15 reduziert.

Es kann aber auch vorgesehen werden, dass die Feder kurz ausgebildet ist und sich an einer im Zylinder 15 hin- und her verschiebbaren Halterung 62 abstützt, die in der gewünschten Stellung des Kolbens 14 im Zylinder 15 arretierbar ist. Dann sind die Stützschalen 20 nur über den kurzen Federweg gegeneinander beweglich, während die Lage der Halterung 62 im Zylinder 15 den Grundabstand der Halteschalen 20 festlegt. Die Halterung 62 und die kürzere Feder 17' sind in der Zeichnung gestrichelt dargestellt.

An den gegenüberliegenden Enden 19 der Federstange 14, 15 ist jeweils eine Stützschale 20 angeordnet. Im Einzelnen ist die Anordnung so getroffen, dass die Stützschalen 20 jeweils über ein Kugelgelenk 21 drehbar und in Richtung der Doppelpfeile 22 verschwenkbar an der Federstange und somit an dem Stellmittel 13 gehalten werden. Die Ausbildung derartiger Kugelgelenke 21 ist grundsätzlich bekannt und bedarf daher keiner weiteren Erläuterung.

Die Stützschalen 20 sind in Richtung auf das zugeordnete Bein 11 offen ausgebildet und haben einen Öffnungswinkel α von 75° bis 120° bezogen auf einem Kreis mit dem Krümmungsradius der Stützschalen. In der Ebene senkrecht zu der Zeichenebene können die Stützschalen 20 eine teilzylindrische und somit gerade Kontur aufweisen. Auch ist es möglich, dass die Stützschalen 20 zusätzlich in Bezug auf die Federstange 14, 15 konvex ausgebildet sind derart, dass sie sich gut an den Oberschenkel 11 anpassen.

Durch die drehbare und schwenkbare Anordnung der Stützschalen an der Federstange 14, 15 einerseits und der teilzylindrischen oder konvexen Ausgestaltung der Halteschalen andererseits liegen die Halteschalen mit ihrer dem Oberschenkel 11 zugewandten offenen Seite 23 gut an dem Oberschenkel 11 an. Es kann vorgesehen werden, dass die dem Oberschenkel 11 zugewandte Oberfläche 24 der Stützschalen 20 mit einem elastischen und weichen Material 25 beschichtet ist. Vorzugsweise ist dieses Material 25 Bestandteil eines Bezugs, der über die Stützschalen 20 gezogen werden kann. Der Bezug umgreift dabei die freien Enden 26 der Stützschalen 20, so dass scharfe Kanten überdeckt werden. Dadurch können Druckstellen und Verletzungen am Oberschenkel vermieden werden.

Weiterhin sind die Stützschalen 20 mit Riemen 27 versehen, die auf der Rückseite 28 der Stützschalen 20 mit diesen verbunden sind. Auf den sich gegenüberliegenden und die Schale begrenzenden Seitenwände der Stützschale ist jeweils ein Riemen 27 angeordnet, wobei die freien Enden 29 der Riemen 27 einer Stützschale mit korrespondierenden Klettverschlüssen versehen sind, um die Riemen um das Bein 11 legen und verschließen zu können. In der Zeichnung sind auf der rechten Seite die Riemen 27 in gelöster Lage und auf der linken Seite in der geschlossenen und angelegten Lage dargestellt. Dadurch ist es mit einfachen Mitteln möglich, die Stützschalen 20 an dem Bein 11 in der gewünschten Lage oberhalb des Knies zu positionieren und zu fixieren. Die Riemen mit Klettverschluss sind dabei leicht zu befestigen beziehungsweise zu lösen. Es können aber auch Knöpfe oder Schnallen an den freien Enden der Riemen 27 angeordnet werden.

Weiterhin kann der Zylinder 15 Arretiermittel 30 aufweisen, die in der eingedrückten und somit wirksamen Lage die Bewegung des Kolbens 14 begrenzen können. Es kann vorgesehen werden, dass bei eingedrücktem Arretiermittel 30 ein weiteres Zusammendrücken der Stange 13 verhindert wird, da die Scheibe 31 mit ihrer dem Kolben 14 abgekehrten Seite an das Arretiermittel anschlägt. Alternativ ist es möglich, ein zu weites Ausfahren des Kolbens 14 aus dem Zylinder zu verhindern, in dem das Arretiermittel 30 erst bei eingedrücktem Kolben 14 betätigt wird. Dann schlägt die Scheibe 31 mit ihrer dem Kolben zugewandten Seite an das Arretiermittel an. Grundsätzlich können aber auch andere Arretiermittel, beispielsweise eine Klemmschraube, vorgesehen werden. Die dargestellten Arretiermittel 30 können auch mit der Halterung 62 zusammenwirken und deren Lage im Zylinder 15 festlegen.

Zum Anlegen des Hygienespreizers wird zunächst der Kolben 14 in den Zylinder gedrückt und dort durch das hintere, in der Zeichnung linke Arretiermittel 30 arretiert. Damit weist die Federstange 14, 15 ihre kürzeste Länge auf. In dieser Lage kann der Hygienespreizer 12 gut zwischen den Oberschenkeln einer Person angelegt werden. Die Stützschalen gelangen zur Anlage an der Innenseite der Oberschenkel 11 und passen sich der jeweiligen Stellung aufgrund des Kugelgelenks 21 an. Die Stützschalen werden dann über die Riemen 27 in der gewünschten Lage am Oberschenkel 11 fixiert. Anschließend kann das Arretiermittel gelöst werden, so dass die Feder 17 den Kolben 14 sanft aus dem Zylinder bewegt. Die Beine 11 der Person werden damit gespreizt, so dass die Innenseite der Oberschenkel und auch der Gesäßbereich für einen Waschvorgang gut erreichbar sind.

Falls während des Waschvorgangs die Person einen Krampf bekommt, durch den die Beine zusammengepresst werden, verhindert der Hygienespreizer 12 ein festes Aneinanderliegen der Beine. Vielmehr bleiben die Beine 11 in der gespreizten Lage, so dass die Person weiterhin gewaschen und gepflegt werden kann. Durch die Federbelastung des Kolbens 14 wird ein sanftes Auffangen der sich zusammenpressenden Beine bewirkt, so dass der zu pflegenden Person keine zusätzlichen Schmerzen entstehen.

Nach dem Waschvorgang werden die Riemen 27 gelöst und der Hygienespreizer 12 kann ohne weiteres wieder entfernt werden. Die Bezüge 25 können dann von den Stützschalen 20 abgezogen und gereinigt werden. Die Stützschalen 20 können zudem lösbar mit dem Stellmittel 13 verbunden sein, so dass der Hygienespreizer mit unterschiedlich großen Stützschalen für unterschiedlich große Beine unterschiedlicher Personen angepasst werden kann. Weiterhin ist der Hygienespreizer einfach und robust ausgebildet, so dass sie auch von ungeübtem Pflegepersonal, beispielsweise in der häuslichen Pflege, angewandt werden kann.

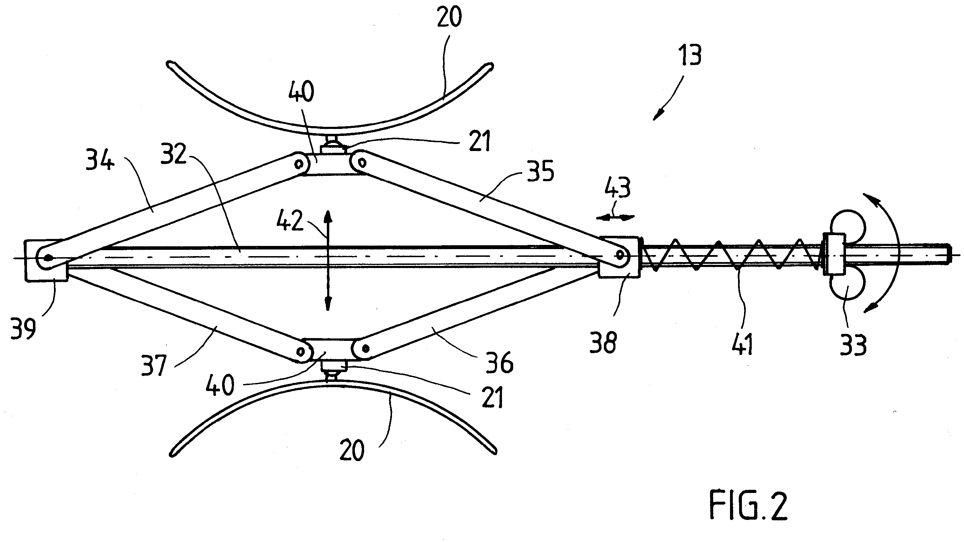

In der Figur 2 ist eine andere Ausführungsform des Hygienespreizers oder Beinspreizers mit einem anderen Stellmittel 13 gezeigt. Hier ist das Stellmittel 13 zum Spreizen der Beine als Scherengelenk ausgebildet, das mit einer zentralen Gewindestange 32 und einer darauf geführten Stellschraube 33 zwischen einer engen Gebrauchslage, bei der die sich gegenüberliegenden Halteschalen 20 einen geringen Abstand zueinander aufweisen, und einer weiten Gebrauchslage, bei der die sich gegenüberliegenden Halteschalen oder Stützschalen 20 einen großen Abstand zueinander aufweisen, in Richtung des Doppelpfeils 42 hin-und herverstellbar ist. In der Figur 2 ist eine mittlere Gebrauchslage dargestellt.

Im Einzelnen ist die Anordnung so getroffen, dass die Scherenarme 34, 35, 36, 37 mit ihrem einen Ende paarweise an einem ersten Block 38 und einem zweiten Block 39 gelenkig verbunden sind. Die Gewindestange 32 erstreckt sich durch beide Blöcke 38, 39 hindurch, und der rechte Block 38 ist in Richtung des Doppelpfeils 43 frei auf der Gewindestange 32 verschiebbar. Im Block 39 ist die Gewindestange 32 axial festgelegt und frei drehbar gelagert. Die anderen Enden der Scherenstangen sind jeweils paarweise mit einer Verbindungsplatte 40 verbunden. Auf den sich gegenüberliegenden und abgewandten Seiten der Verbindungsplatten 40 sind die Stützschalen 20 über Kugelgelenke 21 angebracht.

Die Stellschraube 33 wirkt über eine Druckfeder 41 mit der Block 38 zusammen derart, dass beim Drehen der Stellschraube 32 in Richtung auf den Block 38 dieser in Richtung auf den gegenüberliegenden Block 39 wandert und somit die Scherenarme 34, 35, 36, 37 spreizt, so dass der Hygienespreizer in die weite Gebrauchslage gelangt. Gleichwohl bleibt der Hygienespreizer elastisch derart, dass die Stützschalen 20 entgegen der Kraft der Feder 41 zusammengedrückt werden können. Über die Stellschraube 33 können auch zusammengepresste Beine auseinander bewegt werden.

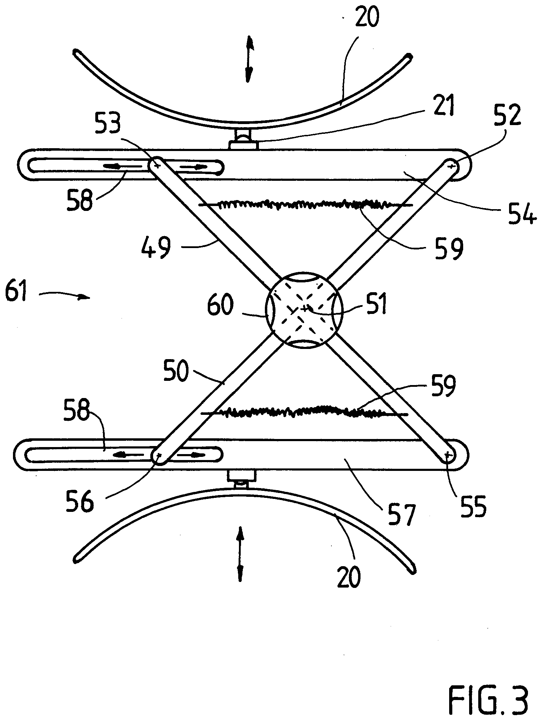

Bei der in der Figur 3 gezeigten Ausführungsform ist das Stellmittel 61 ebenfalls als Scherengelenk ausgebildet. Allerdings bilden die Scherenarme 49, 50 ein Kreuz und sind mittig um eine Drehachse 51 miteinander verbunden. Die freien Enden 52, 53 der Scherenarme 49, 50 sind mit einer ersten Schiene 54 und die gegenüberliegenden freien Enden 55, 56 der Scherenarme sind mit einer zweiten Schiene 57 verbunden. Die Enden 52, 55 der Scherenarme 49, 50 sind drehbar an den Schienen 54, 57 angelenkt. Die Enden 53, 56 der Scherenarme 49, 50 sind drehbar und in Langlöchern 58 verschiebbar an den Schienen 54, 57 gelagert. Somit wird erreicht, dass beim Auseinanderklappen der Scherenarme 49, 50 sich die Schienen 54, 57 parallel zueinander auseinanderbewegen und umgekehrt.

An den Schienen 54, 57 ist jeweils eine Stützschale 20 über ein Kugelgelenk 21 angebracht. Die Stützschalen 20 können somit über das Scherengelenk in die enge und in die weite Gebrauchslage bewegt werden. Es können zwischen den Scherenarmen 49, 50 Zugfedern 59 vorgesehen werden, durch die der Hygienespreizer selbsttätig in die weite Gebrauchslage gebracht und gehalten wird. Anstelle von Zugfedern können auch elastische Zugbänder verwendet werden, die einfacher zu reinigen sind.

Weiterhin wird die Drehachse 51 durch eine Klemmschraube gebildet, so dass die Scherenarme 49, 50 in jeder beliebigen Lage zischen der engen und weiten Gebrauchslage arretiert werden können. Hierzu wird das Handschraubenrad 60 entweder gelöst oder festgezogen. Die Handhabung des Hygienespreizers wird dadurch erleichtert. Der Hygienespreizer wird in der engen Gebrauchslage arretiert und kann so leicht zischen den Oberschenkeln plaziert werden. In dieser Lage können die Stützschalen 20 mit den Oberschenkeln verbunden werden.

In der Zeichnung sind die Stützschalen 20 über die Kugelgelenke 21 unmittelbar mit den Schienen 54, 57 verbunden. Es kann aber auch jeweils eine Zwischenplatte vorgesehen werden, die an den Schienen befestigt ist und die Stützschalen trägt.

Anschließend wird die Handschraubenrad 60 gelöst und die Stützschalen 20 werden durch die Zugfedern 59 über die Scherenarme 49, 50 auseinander bewegt. In der gespreizten Lage kann das Handschraubenrad wieder 60 angezogen werden, so dass die gespreizte Lage sicher aufrechterhalten wird. Bei entsprechend starken Zugfedern ist dies nicht zwingend erforderlich. Durch das Handschraubenrad 60 kann zudem ein gewisser Widerstand eingestellt werden, der zum Zusammendrücken der Stützschalen 20 überwunden werden muss. Dadurch wird erreicht, dass der Patient im Falle einer Spastik nicht gegen einen starren Widerstand drückt.

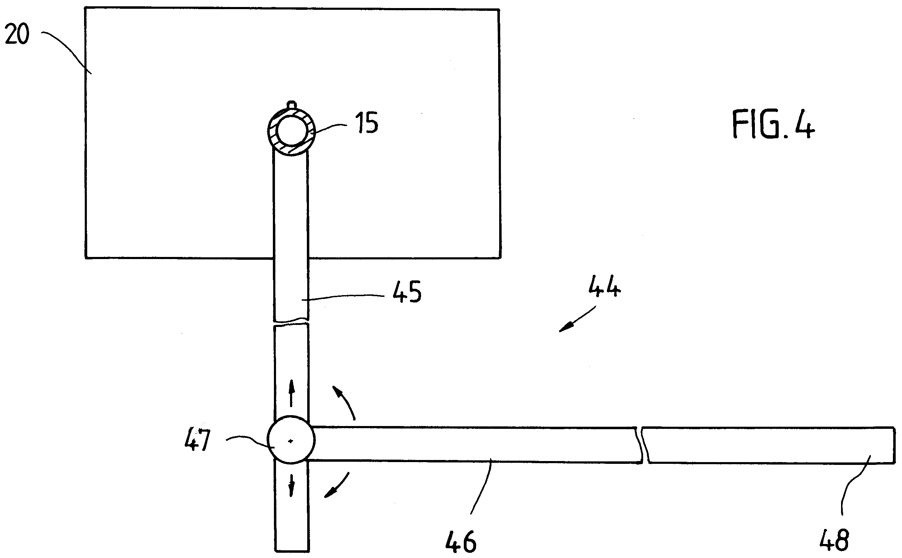

Die Figur 4 zeigt eine weitergehende Ausführungsform des Hygienespreizers gemäß Figur 1. Die Figur 4 zeigt einen Querschnitt durch das äußere Aufnahmerohr 15 der Teleskopstange 14, 15 in Figur 1. Hier ist eine Haltevorrichtung 44 vorgesehen, mit der das Stellmittel 13 beziehungsweise die Teleskopstange 14, 15 an einem Stuhl und insbesondere einem Rollstuhl befestigt werden kann. Die Haltevorrichtung 44 umfasst ein Gestänge, dessen erster Arm 45 mit einem Ende mit dem äußeren Rohr 15 der Teleskopstange verbunden ist. Die Verbindung kann über ein nicht gezeigtes feststellbares Dreh- und Schwenkgelenk erfolgen. Solche Drehgelenke sind allgemein bekannt und bedürfen daher keiner weiteren Erläuterung. Der erste Arm kann aber auch starr mit dem Aufnahmerohr 15 verbunden sein.

An dem anderen Ende des ersten Arms 45 ist ein zweiter Arm 46 des Gestänges über ein feststellbares Dreh-Schiebegelenk 47 mit dem ersten Arm verbunden. Der erste Arm 45 ist somit verschiebbar an dem Gelenk 47 und der zweite Arm 46 drehbar an dem Gelenk 47 gehalten. Solche Dreh-Schiebegelenke sind allgemein bekannt und bedürfen daher ebenfalls keiner weiteren Erläuterung. Das freie Ende 48 des zweiten Arms 46 kann an dem nicht gezeigten Rollstuhl beispielswiese durch eine hülsenförmige Aufnahme befestigt werden, die beispielsweise verschwenkbar unterhalb der Sitzfläche des Rollstuhl gehalten ist. Durch die mehrfach gelenkige Verbindung des Stellmittels 13 beziehungsweise des äußeren Rohrs 15 an dem Rollstuhl können die Stützschalen in eine Position gebracht werden, in der sie gut an den Oberschenkeln der im Rollstuhl sitzenden Personen anliegen. Dann können die Beine während des Sitzens gut auseinander gehalten werden. Vorzugsweise ist das Gestänge 45, 46 lösbar mit dem Hygienespreizer verbunden, so dass dieser auch unabhängig von der Haltevorrichtung 44 verwendet werden kann.

Durch die erfindungsgemäße Vorrichtung ist es möglich, dass nur eine Person eine zu Spastiken neigende Person gut pflegen und waschen kann. Die Anwendung ist harmlos und stellt keine Gefährdung der pflegebedürftigen Person dar. Sie kann leicht angelegt und auch wieder abgelegt werden. Weiterhin kann vorgesehen werden, dass dieser Hygienespreizer beispielsweise in häuslichem Bereich der zu pflegenden Person vorhanden ist oder durch das Pflegepersonal mitgeführt wird. In jedem Fall sind eine Pflege und ein Waschen der betreffenden Peron einfach und in relativer kurzer Zeit möglich.

Aufgrund der Bewegungsmöglichkeit der Halteschalen gegeneinander über das federnde Stellmittel kann die Vorrichtung auch zu therapeutischen Zwecken eingesetzt werden. Hierfür ist es günstig, wenn die Feder über den gesamten Weg zwischen der engen und der weiten Gebrauchslage wirksam ist. Dann kann die Adduktion der Oberschenkel geübt werden.

Auch kann die Vorrichtung zur Übung der Abduktion eingesetzt werden, in dem die Schalen mit Riemen an den Oberschenkeln gehalten werden und die Feder als Zugfeder ausgebildet ist. Hierfür müsst lediglich die Feder ausgetauscht werden. Dann müssen für Übungszwecke die Beine entgegen der Zugkraft der Feder auseinander bewegt werden. Es kann auch vorgesehen werden, dass die Halteschalen über ein gebogenes Gestänge auf die andere Seite des Oberschenkels oder Unterschenkels geführt werden und auf der Außenseite der Schenken anliegen.

## Patentansprüche

1. Hygienespreizer zum Auseinanderhalten der Beine eines Menschen insbesondere während der Pflege, **dadurch gekennzeichnet, dass** der Hygienespreizer an seinen gegenüberliegenden Enden jeweils eine Stützschale (20) aufweist, die in der Gebrauchslage jeweils an der Innenseite eines Beins (11) des Menschen anliegt, und dass zwischen den zugwandten Seiten der Stützschalen ein Stellmittel vorhanden ist, um die Stützschalen zwischen einer engen Gebrauchslage, in der die Stützschalen einen kleinen Abstand zueinander aufweisen, und einer weiten Gebrauchslage, in der die Stützschalen einen großen Abstand zueinander aufweisen, hin- und her zu bewegen, wobei die Stützschalen entgegen der Kraft einer Feder (17, 41) von einer weiten Gebrauchslage in Richtung auf die enge Gebrauchslage bewegbar sind.

2. Hygienespreizer nach Anspruch 1, **dadurch gekennzeichnet, dass** zumindest die dem Bein (11) zugewandte Seite der Stützschalen (20) mit einem elastischen und/oder weichen Material (25) versehen, beschichtet oder beziehbar ist.

3. Hygienespreizer nach Anspruch 1 oder 2, **dadurch gekennzeichnet, dass** eine oder beide Stützschalen (20) um wenigstens eine Achse gelenkig und insbesondere über Kugelgelenke mit dem Stellmittel (13) verbunden sind.

4. Hygienespreizer nach einem der Ansprüche 1 bis 3, **dadurch gekennzeichnet, dass** das Stellmittel (13) in der voll ausgefahrenen Position, die der weiten Gebrauchslage der Stützschalen entspricht, und/oder der voll eingefahrenen Position, die der engen Gebrauchslage der Stützschalen entspricht, und/oder in mehreren Positionen zwischen der ausgefahrenen und eingefahren Position arretierbar ist.

5. Hygienespreizer nach einem der Ansprüche 1 bis 4, **dadurch gekennzeichnet, dass** der Abstand der Stützschalen (20) in der engen Gebrauchslage zwischen 7 cm und 20 cm und in der weiten Gebrauchslage zwischen 20 cm und 60 cm beträgt.

6. Hygienespreizer nach einem der Ansprüche 1 bis 5, **dadurch gekennzeichnet, dass** das Stellmittel (13) eine Teleskopstange (14, 15) umfasst, die als Federstange ausgebildet ist derart, dass die Stützschalen (20) entgegen der Kraft einer Feder (17) zusammendrückbar sind und aufgrund der Federkraft in der weiten Gebrauchslage gehalten werden.

7. Hygienespreizer nach einem der Ansprüche 1 bis 6, **dadurch gekennzeichnet, dass** das Stellmittel (43) als Scherengelenk ausgebildet ist, dessen Scherenarme (34, 35, 36, 37) über eine Stellschraube (32, 33) zwischen der ausgefahrenen und eingefahrenen Position hin- und herbewegbar sind.

8. Hygienespreizer nach Anspruch 7, **dadurch gekennzeichnet, dass** die Stützschalen (20) an der Verbindungstelle (40) an den auseinander gehenden Enden der Scherenarme (34, 35, 36, 37) angelenkt sind.

9. Hygienespreizer nach einem der Ansprüche 1 bis 8, **dadurch gekennzeichnet, dass** das Stellmittel (61) als Scherengelenk mit zwei Scherenarmen (49, 50) ausgebildet ist, die über eine Drehachse (51) unter Bildung eines Kreuzes drehbar miteinander verbunden sind, und dass die einen freien Enden (52, 55) zumindest drehbar und die anderen freien Enden (53, 56) drehbar und verschiebbar an Schienen (54, 57) gelagert sind, an denen die Stützschalen (20) angeordnet sind.

10. Hygienespreizer nach Anspruch 9, **dadurch gekennzeichnet, dass** die Scherenarme (49, 50) durch wenigstens eine Zugfeder (59) in der gespreizten Lage gehalten sind, um die Stützschalen (20) in der weiten Gebrauchslage zu halten.

11. Hygienespreizer nach Anspruch 9 oder 10, **dadurch gekennzeichnet, dass** die Drehachse (51) der Scherenarme (49, 50) als Feststellschraube mit einem Handschraubenrad (60) ausgebildet ist, um die Scherenarme in einer beliebigen Winkelstellung zu arretieren.

12. Hygienespreizer nach einem der Ansprüche 1 bis 11, **dadurch gekennzeichnet, dass** die Feder (17') auf einer Halterung (62) am Stellmittel hin- und herbewegbar gehalten ist.

13. Hygienespreizer nach einem der Ansprüche 1 bis 12, **dadurch gekennzeichnet, dass** das Stellmittel über eine Haltevorrichtung (44) an einem Stuhl des Menschen befestigbar ist.

14. Hygienespreizer nach Anspruch 13, **dadurch gekennzeichnet, dass** die Haltevorrichtung ein Gestänge umfasst, deren einzelne Stangen (45, 46) über feststellbare Gelenke (47) miteinander verbunden sind.

15. Hygienespreizer nach Anspruch 14, **dadurch gekennzeichnet, dass** die Gelenke (47) auf den Stangen (45) verschiebbar gelagert sind.
